# EUROPEAN PATENT SPECIFICATION

(11) **EP 2 232 265 B1**
(45) Date of publication and mention of the grant of the patent: **18.05.2016**
(21) Application number: 08733527.9
(22) Date of filing: 29.04.2008
(51) Int. Cl.: G01N 33/53, G01N 33/543, G01N 33/58, G01N 33/68, G01N 33/536

(54) **METHOD FOR QUANTIFYING ALLERGEN-ESPECIFIC HUMAN IGG SUBCLASSES FOR THE CONTROL AND ATTENDANCE OF SPECIFIC IMMUNOTHERAPY**
VERFAHREN ZUR QUANTIFIZIERUNG ALLERGENSPEZIFISCHER MENSCHLICHER IGG-UNTERKLASSEN ZUR KONTROLLE UND BETREUUNG EINER SPEZIFISCHEN IMMUNTHERAPIE
PROCÉDÉ POUR QUANTIFIER DES SOUS-CLASSES D'IGG HUMAINES SPÉCIFIQUES D'ALLERGÈNES POUR LE CONTRÔLE ET LE SOIN D'IMMUNOTHÉRAPIE SPÉCIFIQUE

(30) Priority: 02.01.2008 BR 0800612
(43) Date of publication of application: 29.09.2010
(73) Proprietor: Fundacao De Amparo A Pesquisa Do Estado De Minas Gerais - Fapemig, 30330-080 Minas Garais (BR); Universidade Federal De Uberlândia - Ufu, 38401-136 Minas Gerais (BR)
(72) Inventor: TAKETOMI, Ernesto Akio, Uberlandia 38400-902 Minas Gerais (BR); SILVA, Deise Aparecida de Oliveira, Uberlandia 38400-902 Minas Gerais (BR)
(74) Representative: Böck, Bernhard
(86) International application number: PCT/BR2008/000124
(87) International publication number: WO 2009/082798

(56) References cited:
- EP-A2- 0 124 103
- EP-A2- 0 161 868
- WO-A1-90/13817
- WO-A2-2006/047670
- JP-A- 8 062 217
- US-A- 4 256 833
- US-A1- 2006 008 895
- DATABASE WPI Derwent Publications Ltd., London, GB; AN 1996-191810 THOMPSON & JP 8 062 217 A
- HONG CS ET AL.: 'Measurement of IgE and IgG subclass antibodies to whole body antigen and two major allergens (Der fl & Der fll) of Dermatophagoides farinae in normal subjects and asthmatics.' YONSEI MED J. vol. 35, no. 4, December 1994, pages 453 - 63
- JIMENO L ET AL.: 'Monoclonal antibody-based ELISA to quantify the major allergen of Artemisia vulgaris pollen, Art v 1.' ALLERGY. vol. 59, no. 9, September 2004, pages 995 - 1001

## Description

### FIELD OF THE INVENTION

The present invention relates to a method for quantifying allergen specific human IgG subclasses, including IgG1, IgG2, IgG3 and IgG4 subclasses, in serum samples or other biological fluids from patients with allergic respiratory disease, through a reverse ELISA immunoenzymatic technique, specific against allergens used in allergen-specific immunotherapy; The present invention also relates to a kit comprising microtitration plates for ELISA previously sensitized/coated with monoclonal antibodies, for example, anti-Der p 1 or anti-Der p 2, corresponding allergens' total extract, labeled secondary antibodies, enzymatic conjugate, enzymatic substrate and chromogenic buffer, as well as the positive and negative reference control sera.

### BACKGROUND OF THE INVENTION

Allergen-specific immunotherapy, also called treatment with allergy vaccines, is indicated for atopic patients with IgE mediated allergic diseases, for example, allergic rhinitis, light or moderate asthma and hymenoptera sting allergy. It involves the administration of gradually increasing levels of specific allergens until an effective dose that enables the reduction of the severity of the disease, even in the presence of the natural allergen, is reached., even maintaining natural exposition to the allergen.

The increase in IgG antibodies is dose-dependent and occurs after administration of a sufficient amount of the allergen. Immunological response involves the production of IgG1 subclass antibodies in the initial phase of specific immunotherapy, and IgG4 subclass is the dominant subclass in prolonged specific immunotherapy. With continuing immunotherapy, allergen-specific IgG levels tend to increase until stabilize in a plateau. IgG antibodies induced by allergen immunotherapy may act as allergen blocking antibodies. This theory of blocking antibodies postulates that IgG antibodies compete with IgE antibodies for allergen biding, blocking IgE dependent mast cell activation.

Currently, there is no routine laboratorial test for allergen-specific IgG antibodies detection, particularly IgG1 and IgG4 subclasses. Physicians, who assist patients with respiratory allergy that have been submitted to allergen-specific immunotherapy, are following the treatment of such patients only through clinic parameters which are very subjective. The possibility of following such patients using IgG1 and/or IgG4 antibodies dosage, specific to certain allergens, will enable to follow those patients through objective parameters in association with subjective clinical parameters.

The possibility of following patients with respiratory allergies, such as rhinitis and asthma, submitted to allergen-specific immunotherapy through the laboratory evaluation of specific IgG antibodies levels, has stimulated researchers to develop objective methods for quantifying those specific IgG antibodies.

Researchers have developed the conventional ELISA technique, which uses allergens directly bound to ELISA microtitration plates, as described in the international patent application PCT/FR90/00335, where allergen-specific IgG4 antibodies present in the test sample, for example, serum samples, are captured and later detected using a non-human monoclonal antibody anti human IgG4. Detection is achieved by a labeled antibody against the non-human epitope part of the monoclonal antibody. This technique presents the important disadvantage of requiring the existence of purified allergens or antigens for the detection of the corresponding IgG antibodies, which are often too expensive or difficult to obtain in a purified and isolated form.

Additionally, researchers have also developed immunoenzymatic techniques such as the reverse-sandwich ELISA, where the sandwich is made of two layers of antigens, with an antibody layer in between. It has been described that this technique presents higher sensitivity and specificity when compared to the indirect conventional ELISA (Miyazawa, H. et al., Journal of Allergy and Clinical Immunology, 82 (3): 407-413; Miyazawa, H. et al., Clinical and Diagnostic Laboratory Immunology, 6 (5): 701-704, 1999).

However, this technique presents the inconvenience of requiring the use of specific equipments to perform the reading of the assay, as shown in the patent document PI 9400447-1. This association, in which a given assay requires the use of specific equipments, allows for a direct dependence between the producers of the diagnostic kits and of the required equipments and the consumers. Often consumers, in this case analytical laboratories, will be required to consume a minimal monthly quota of the kits produced by a given company in order to be able to use the necessary equipment. Thus, this requirement for a specific equipment in order to perform the assays leads to an increase in price of the final product.

The present invention presents the important advantage of allowing for the realization of the tests without the need for special laboratory facilities and/or the acquisition of exclusive and costly equipments, which therefore, makes the assay more accessible.

In 2001, the inventors and collaborators of the present invention developed a reverse ELISA technique for quantifying Der p 2 allergen-specific IgE antibodies, using recombinant Der p 2 allergens and/or Der p 2 specific monoclonal antibodies. This technique was developed with the intention of aiding allergy diagnosis, having in mind that the presence of Der p 2 allergen-specific IgE antibodies indicate the occurrence of an allergic response in the patient. It has also been demonstrated that this technique has a higher sensitivity related to conventional ELISA (Silva et al., Annals of Allergy, Asthma, Immunology, 86 (5): 545-550, 2001; PI 0116933-5).

On the other hand, the detection IgG antibodies or particularly IgG1 and IgG4 subclasses, specific against Derp 2, or against any other specific allergen, would indicate the development of patient's physiological response, i.e., a defense response against dust mite allergens.

Thus, having in mind what was described above, it becomes particularly interesting to development a method and a kit for quantifying IgG antibody subclasses specific against clinically relevant allergens. These antibodies can be detected in the serum or other biological fluids such as saliva from patients with allergic respiratory disease using a reverse ELISA immunoenzymatic technique and relevant monoclonal antibodies for the control and attendance of allergen-specific immunotherapy.

In this way, this assay represents a potential tool for control and attendance of patients with respiratory allergy, especially during immunotherapy with allergy vaccines. This technique has been shown to be more sensitive than conventional ELISA as well as being an alternative method which is much more accessible than traditional quimioluminescence techniques.

### SUMMARY OF THE INVENTION

The present invention aims to provide a method for quantifying allergen-specific IgG antibody subclasses, particularly of IgG1, IgG2, IgG3 and IgG4, for the control and attendance of allergen-specific immunotherapy in patients with allergic respiratory disease, using a reverse ELISA immunoenzymatic technique. In this technique, allergens are, preferentially derived from *Dermatophagoides pteronyssinus* dust mite, more specifically Der p 1 or Der p 2, which are captured using anti-Der p 1 or anti-Der p 2 monoclonal antibodies, respectively, which in turn, are bound to ELISA microtitration plates.

Additionally, the present invention aims to provide a kit comprising ELISA microtitration plates previously sensitized/coated with monoclonal antibodies, for example, anti-Der p 1 or anti-Der p 2, corresponding allergens' total extract, secondary labeled antibodies, enzymatic conjugate, enzymatic substrate and chromogenic buffer, as well as the reference positive and negative control sera.

The present invention also aims to demonstrate that such method provides higher sensitivity in the detection of different subclasses of specific IgG antibodies for the control and attendance of allergen-specific immunotherapy in patients with allergic respiratory disease.

### BRIEF DESCRIPTION OF THE DRAWINGS

The following figures are part of the present specification and are herein included in order to illustrate specific aspects of the invention. The object of the present invention may be better understood with reference to one or more of those figures, in association with the detailed description of the preferred embodiment herein presented.
FIG. 1
Shows a representative scheme of the reverse ELISA immunoenzymatic technique, wherein:
   1 - Anti-allergen capture antibody;
   2 - Total extract;
   3 - Specific allergen;
   4 - IgG antibody;
   5 - Biotinylated anti-IgG detection antibody; and
   6 - Streptavidin-Peroxidase.

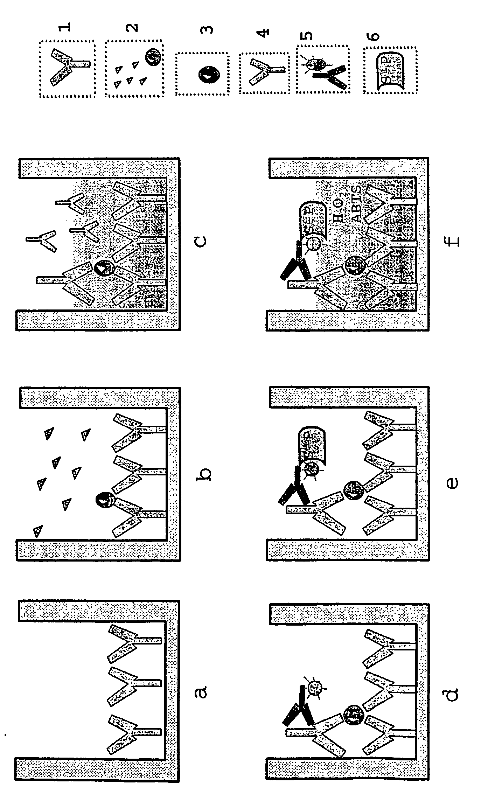

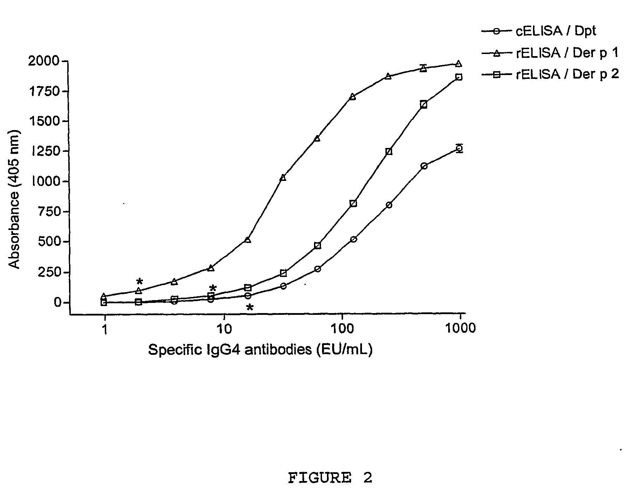

FIG. 2 Shows standard curves of conventional ELISA technique (cELISA) for the quantification of IgG4 antibodies specific against total *D. pteronyssinus* (Dpt) extract, and reverse ELISA (rELISA) for the quantification of IgG4 antibodies specific against Der p 1 or Der p 2. Assay's sensitivities are indicated by asterisks (*): 15.6 EU/mL for cELISA/Dpt, 1.95 EU/mL for rELISA/Der p 1, and 7.8 EU/mL dor rELISA/Der p 2.

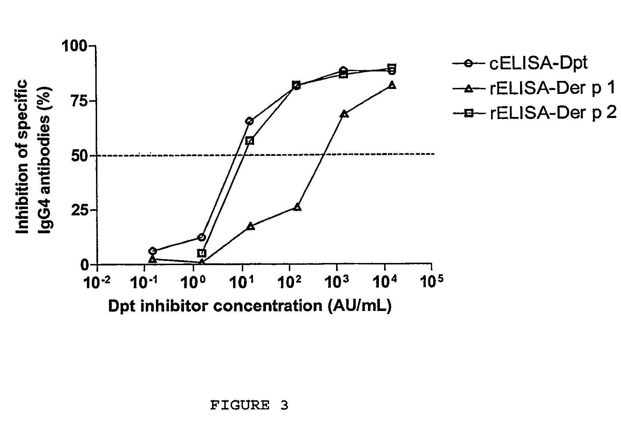

FIG. 3 shows inhibition tests for the quantification of IgG antibodies specific against total *D. pteronyssinus* (Dpt) extract and Der p 1 and Der p 2 by by cELISA and rELISA, respectively. Total *D. pteronyssinus* extract, inhibitor, was submitted to serial 10-fold dilutions from 0.15 to 15,000 Allergenic Unity per milliliter (AU/mL) in ELISA buffer. Each extract dilution was mixed in a proportion of 1:1 with a pool of reference sera containing specific IgG4 antibodies and incubated at 37°C during two hours. As a positive control, the reference sera were incubated only with ELISA buffer without the inhibitor. IgG4 antibodies specific against total Dpt extract and Der p 1 and Der p 2 allergens were determined by measuring the absorbance obtained by the respective ELISA assays. The inhibition percentage was calculated as follows: 100 - [(test sample absorbance with inhibitor / positive control absorbance without inhibitor) x 100].

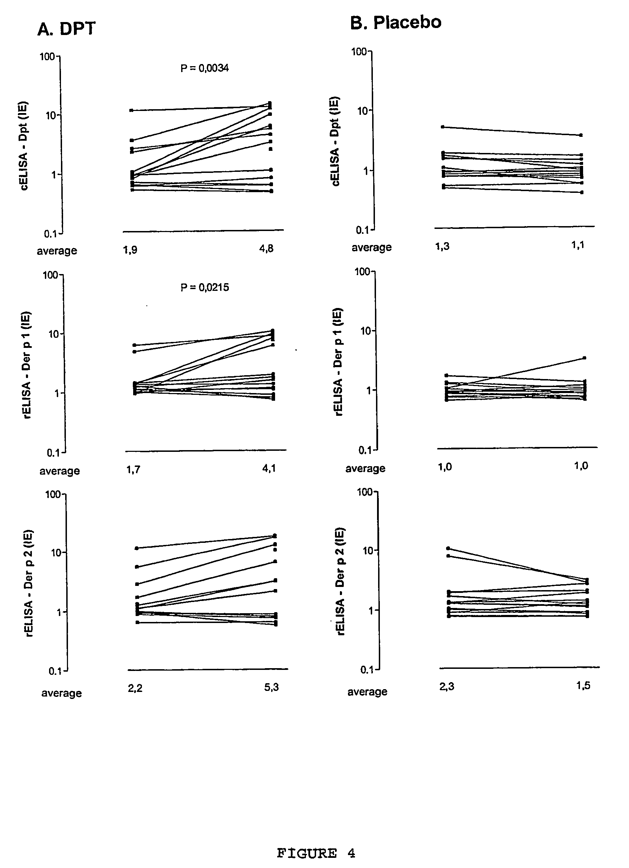

FIG. 4 Shows the levels of IgG4 antibodies specific against total *D. pteronyssinus* (Dpt) extract determined by cELISA, and IgG4 antibodies specific against the principal allergens Der p 1 and Der p 2 determined by rELISA, in serum samples of 15 patients allergic to dust mite under specific immunotherapy with *D. pteronyssinus* extract, A: DPT group and 15 patients without active immunotherapy, B: placebo group. Antibody levels are expressed in ELISA index (EI) as values of individual patients at two different times, i.e., day 0 and after one year of immunotherapy and connected by a line. Arithmetic averages of EI values are also indicated. Statistically significant differences before and after immunotherapy within each groups were determined by the Wilcoxon test (P < 0.05).

### DETAILED DESCRIPTION OF THE INVENTION

The present invention aims to provide a method and a kit for quantifying levels of allergen-specific IgG antibody subclasses, particularly of IgG1, IgG2, IgG3 and IgG4 subclasses, in serum samples or other biological fluids such as, for example, saliva using a reverse ELISA immunoenzymatic technique as a potential tool for the control and attendance of allergen-specific immunotherapy in patients with allergic respiratory disease.

Specific anti-allergen monoclonal antibodies, such as, for example, anti-Der p 1 or anti-Der p 2, are bound to ELISA microtitration plates in order to capture the corresponding allergens, Der p 1 or Der p 2, respectively, present in crude *Dermatophagoides pteronyssinus* extract which, subsequently, interacts with specific IgG antibodies in serum samples or other biological fluids from allergic patients.

Those antibodies are later detected by the addition of labeled polyclonal human antibodies, against IgG subclasses preferentially IgG1, IgG2, IgG3 or IgG4, labeled with, for example, biotin and, subsequently with an enzymatic conjugate such as, for example, streptavidin-peroxidase. Reaction revelation is achieved by the addition of an enzymatic substrate, for example, hydrogen peroxide diluted in a chromogenic buffer such as, for example, 2,2'-azino-bis(3-ethylbenzthiazoline-6-sulphonic acid) - ABTS, and absorbance is determined in a microtitration plate reader, for example, at 405 nm.

According to the present invention, the method for quantifying allergen-specific IgG subclasses antibodies, using a reverse ELISA immunoenzymatic technique comprises the the following steps:
▪ Sensitization of microtitration plates for high affinity ELISA with mouse monoclonal antibodies against specific allergen specific;
▪ Blocking of the active sites of the microtitration plates with phosphate buffered saline solution (PBS) added to Tween 20 surfactant and bovine serum albumin (PBS-T-BSA);
▪ Addition of total extract of the corresponding allergen;
▪ Incubation with serum samples or other biological fluids from allergic patients;
▪ Addition of human labeled secondary polyclonal antibodies anti-IgG1, anti-IgG2, anti-IgG3 or anti-IgG4;
▪ Addition of an enzymatic conjugate;
▪ Addition of an enzymatic substrate diluted in a chromogenic buffer;
▪ Determination of absorbance in a microtitration plate reader; and
▪ Expression of results in EU/mL compared to a standard curve obtained using reference human sera.

According to the present invention, in the step concerning sensitization of microtitration plates for high affinity ELISA, anti-Der p 1 mouse monoclonal antibody clones can be used. Monoclonal antibodies against different dust mite allergens such as Der p 2, Der f1, Der f 2, Blo t 5; against fungus such as Asp f 1; against dog such as Can f 1; against cat such as Fel d 1; against cockroach such as Bla g 1 and Bla g 2, and other immunodominant substances can also be used.

Preferentially, mouse monoclonal antibodies against specific allergens for sensitization of microtitration plates for high affinity ELISA are anti-Der p 1, 5H8 clone or others, or anti-Der p 2, 1D8 clone or others.

According to the present invention, in the step involving the sensitization of microtitration plates for high affinity ELISA, 20 to 200 µL/well of mouse monoclonal antibody against specific allergens are added in a protein concentration of 0.5 to 5 µg/well, diluted in 0.01 M to 0.1 M of carbonate-bicarbonate buffer, pH 7.0 to 12.0, and incubated during 6 to 24 hours at a temperature of 2 to 12°C. Preferentially, 50µL/well of the reference monoclonal antibody should be added in a protein concentration of 1µg/well, diluted in 0.06 M of carbonate-bicarbonate buffer, pH 9.6, and incubated during 18 hours at 4°C.

As a control of the mouse monoclonal antibody, the plates were sensitized in parallel with purified mouse IgG in a protein concentration of 0.5 to 50 µg/mL, Preferentially 10 µg/mL.

According to the present invention, in the step concerning blocking of the active sites of microtitration plates with phosphate buffered saline solution (PBS) added to Tween 20 surfactant and bovine serum albumin (PBS-T-BSA, the active sites are blocked through the addition of 50 to 300 µL/well of PBS-T followed by the addition of 0.5 to 5% bovine serum albumin (BSA) and incubated during 30 minutes to 4 hours a temperature of 20°C to 40°C. Preferentially, 200 µL of 1% PBS-T-BSA are added to each well (200 µL/well) and incubated for 1 hour at room temperature or at 37°C.

Additionally, the present invention foresees that different total extracts of dust mite, fungus, dog, cat or cockroach may be utilized in the step concerning the addition of total extract from the corresponding allergen. Preferentially, the total extract is from *Dermatophagoides pteronyssinus.*

According to the present invention in the step concerning addition of total extract from the corresponding allergen, the wells are incubated with 20 to 200 µL/well of total extract from the corresponding allergen in a protein concentration of 10 to 100 µg/mL or of 100 to 5,000 Allergenic Unity per milliliter (AU/mL), during 30 minutes to 4 hours at a temperature of 20°C to 40°C. Preferentially, 50 µL/well of the total extract from the corresponding allergen are added in a concentration of 1,500 AU/mL or 40 µg/mL and incubated during 1 hour at room temperature or at 37°C.

According to the present invention, in the step concerning the incubation with serum samples or other biological fluids from allergic patients, the plates are incubated with 25 to 100 µL/well of samples from allergic samples, primary antibodies, in dilutions up to 1:20, during 30 minutes to 4 hours at a temperature from 20°C to 40°C. Preferentially, 50 µL/well of serum samples from allergic patients are added, in dilutions of 1:2 and 1:5, and incubated during 1 hour at room temperature or at 37°C.

According to the present invention, in the step concerning the addition of human labeled secondary polyclonal antibodies anti-IgG1, anti-IgG2, anti-IgG3 or anti-IgG4, the label is selected from a group consisting of, but not limited to biotin or peroxidase. Preferentially, the label used in this step is biotin.

According to the present invention, in the step concerning the addition of human labeled secondary polyclonal antibodies anti-IgG1, anti-IgG2, anti-IgG3 or anti-IgG4, 25 to 200 µL/well of human biotinylated secondary antibodies anti- IgG1, IgG2, IgG3 or IgG4, preferentially produced in goat, are added to the wells, in a dilution of 1:100 to 1:6000, and the plates are incubated during 30 minutes to 4 hours at a temperature of 20°C to 40°C. Preferentially, 50 µL/well of human biotinylated anti- IgG4 polyclonal antibody are added in a dilution of 1:3000 and the plates are incubated during 1 hour at room temperature or at 37°C.

Additionally, the present invention foresees the addition of an enzymatic conjugate, preferentially of the streptavidin-peroxidase conjugate. According to the present invention, in the step concerning the addition of an enzymatic conjugate, 25 to 200 µL/well of streptavidin-peroxidase are added to the wells in a dilution of 1:100 to 1:300, and the plates are incubated during 15 minutes to 4 hours at a temperature of 20°C to 40°C. Preferentially, 50 µL/well of streptaviidin-peroxidase are added in a dilution of 1:500 and the plates are incubated during 30 minutes at room temperature or at 37°C.

According to the present invention, in the step concerning the addition of an enzymatic substrate diluted in a chromogenic buffer, the enzymatic substrate is, preferentially, hydrogen peroxide, and the chromogenic buffer is selected from a group consisting of, but not limited to 2,2'-azino-bis (3-ethylbenzthiazoline-6-sulphonic acid) (ABTS), orthophenilenediamine (OPD) or tetramethylbenzidine (TMB). Preferentially, the chromogenic buffer used is ABTS.

According to the present invention, in the step concerning the addition of an enzymatic substrate diluted in a chromogenic buffer, 25 to 200 µL/wells of the enzymatic substrate hydrogen peroxide are added in a concentration of 0.001 to 0.1 M diluted in 0.01 to 0.1 M citrate-phosphate buffer, pH 4.0 to 5.0. Preferentially, 50 µL/well of 0.03% enzymatic substrate hydrogen peroxide are added in 0.01 M ABTS chromogenic buffer diluted in 0.07 M citrate-phosphate buffer, pH 4.2.

Additionally, the present invention foresees that different wavelengths (405, 450 or 492 nm) may be used in the step concerning! the determination of absorbance in a microtitration plate reader, depending of the chromogenic buffer employed in the step involving the addition of an enzymatic substrate diluted in a chromogenic buffer previously described. Preferentially, the wavelength used is 405 nm.

According to the present invention, in the step concerning the expression of results in EU/mL compared with a standard curve using reference human sera, the standard curve can be obtained using known quantities of IgG antibody subclasses, particularly IgG1, IgG2, IgG3 or IgG4 specific against purified immunodominant allergens, or IgG antibodies, more specifically chimeric IgG1, IgG2, IgG3 or IgG4 constituted by human Fc gama chains conjugated to Fab portions, an antigen binding fragment, immunodominant allergen produced in animals, preferentially mice.

According to an embodiment of the present invention, in the step concerning the expression of results in EU/mL compared with a standard curve using reference human sera, the standard curve can be obtained using known quantities of purified IgG antibodies specific against Der p 1 or Der p 2 or anti-Der p 2 chimeric IgG antibodies constituted by human Fc gama chains conjugated to Fab portions, an antigen binding fragment, produced in mice.

Results are expressed as ELISA unity per milliliter (EU/mL) and compared to a standard curve obtained in parallel through the measurement of levels of IgG4 antibodies specific against dust mite allergens, using a pool of reference sera containing corresponding specific IgG4 antibodies which were defined as containing 1000 EU/mL of IgG4 antibodies specific against Der p 1 or Der p 2. The standard curve is included in each plate with samples in serial double dilutions varying from 0.5 to 500 EU/mL, in duplicate.

Alternatively, results can be expressed in ELISA index (EI) based in the following formula: EI = sample OD / *cut off*, where the *cut off* is established as the average OD of negative control sera for antibodies of the IgG4 subclass, added by 3 standard deviations.

According to the present invention the method described above is suited for the control of the evolution and attendance of patients with respiratory allergy, especially during allergen-specific immunotherapy treatment, i.e., with allergy vaccines. Besides, this method has been shown more sensitive than conventional ELISA, as it will be subsequently described.

Additionally, the! present invention provides a kit for quantifying allergen-specific IgG antibody subclasses, particularly IgG1, IgG2, IgG3 or IgG4 subclasses, for the control and the attendance of patients with allergic respiratory disease, according to the method described above.

According to the present invention, the kit for quantifying allergen-specific IgG antibody subclasses comprises:
▪ Microtitration plates for high affinity ELISA already sensitized/covered with mice monoclonal antibodies against specific allergens;
▪ Container comprising phosphate buffered saline solution (PBS) with added Tween 20 surfactant and 0.5 to 5% bovine serum albumin (PBS-T-BSA);
▪ Container comprising total extract from the corresponding allergen;
▪ Containers! comprising human labeled secondary polyclonal antibodies anti-IgG1., anti-IgG2, anti-IgG3 or anti-IgG4;
▪ Container comprising an enzymatic conjugate; and
▪ Containers comprising an enzymatic substrate and a chromogenic buffer.

According to the present invention, the kit for quantifying allergen-specific IgG antibody subclasses additionally refers to containers comprising standard reference positive and negative sera.

According to the present invention, anti-Der p 1 mouse monoclonal antibody clones or monoclonal antibodies against different dust mite allergens such as Der p 2, Der f1, Der f 2, Blo t 5; against fungus such as Asp f 1; against dog such as Can f 1; against cat such as Fel d 1; against cockroach such as Bla g 1 and Bla g 2, and other immunodominant substances can be used in sensitization of microtitration plates for high affinity ELISA.

Preferentially, the mouse monoclonal antibodies against specific allergens used for sensitization/covering of microtitration plates for high affinity ELISA are anti-Der p 1, 5H8 clone or others, or anti-Der p 2, 1D8 clone or others.

Additionally, the present invention foresees that different corresponding total extracts of dust mite, fungus, dog, cat or cockroach may be used in the container comprising total extract from the corresponding allergen. Preferentially, the total extract is from *Dermatophagoides pteronyssinus.*

According to the present invention, in the item concerning containers comprising labeled human secondary polyclonal antibodies anti-IgG1, anti-IgG2, anti-IgG3 or anti-IgG4, the label is selected from the group consisting of, but not limited to biotin or peroxidase. Preferentially, the label used in this item is biotin.

Additionally, the present invention foresees that the enzymatic conjugate used in the item concerning a container comprising an enzymatic conjugate is, preferentially, streptavidin-peroxidase.

According to the present invention, in the item concerning a container comprising an enzymatic substrate and a chromogenic buffer, the enzymatic substrate is, preferentially, hydrogen peroxide, and the chromogenic buffer is selected from the group consisting of, but not limited to 2,2'-azino-bis(3-ethylbenzthiazoline-6-sulphonic acid) (ABTS), orthophenilenediamine (OPD) or tetramethylbenzidine (TMB). Preferentially, the chromogenic buffer used is ABTS.

Below is presented a more detailed example of the preferred realization of the present invention. The proposed procedures and data presented constitute representative examples of the present invention and its applications and are not to be interpreted as limitation indicatives of its use.

### EXAMPLE I

Microtitration plates for high affinity ELISA were sensitized by the addition of 50 µL/well of mouse monoclonal antibody against specific allergens, for example, anti-Der p 1, 5H8 clone, or Der p 2, 1D8 clone, in a protein concentration of 1 µg/well, diluted in a 0.06 M carbonate-bicarbonate buffer, pH 9.6, during 18 hours at 4°C.

As a control of the mouse monoclonal antibody, the plates were sensitized in parallel with purified mouse IgG in a protein concentration of 10 µg/mL.

The plates were submitted to 2-6 washing cycles, preferentially 3 cycles with 0.1 M phosphate buffered saline (PBS), pH 7.2, containing 0.01 to 0.2% Tween 20, preferentially 0.05% (PBS-T).

Subsequently, active sites were blocked through the addition of 200 µL/well of PBS-T with 1% bovine serum albumin (BSA), during 1 hour at room temperature or at 37°C.

Subsequent steps were performed using 0.5 to 5% PBS-T-BSA, preferentially 1%, as a diluent, ELISA buffer.

After blocking, the wells were washed as described above and subsequently incubated with 50 µL/well of total extract from the corresponding allergen, for example, *Dermatophagoides pteronyssinus* in a concentration of 1,500 AU/mL or 40 µg/mL, during 1 hour at room temperature or at 37°C.

The plates were submitted to 3-10 washing cycles, preferentially 5 washing cycles, with PBS-T and incubated with 50 µL/well of serum samples of allergic patients, primary antibodies in dilutions of 1:2 and 1:5, during 1 hour at room temperature or at 37°C.

After washing as described above, human biotinylated anti- IgG4 secondary antibody diluted to 1:3000 was added to the wells at 50 µL/well, and the plates were incubated for 1 hour at room temperature or at 37°C.

After a novel washing cycle streptavidin-peroxidase conjugate diluted to 1:500 was added at 50 µL/well and incubated during 30 minutes at room temperature or at 37°C.

After a final washing cycle, the reaction was developed by the addition of 50 µL/well of enzymatic substrate consisting of 0.03% hydrogen peroxide in a 0.01 M ABTS chromogenic buffer diluted in a 0.07 M citrate-phosphate buffer, pH 4.2.

Reading of the reaction results was performed by the determination of the optical density (OD) in a microtitration plate reader for ELISA at 405 nm.

### COMPARISON BETWEEN rELISA AND cELISA

The reverse ELISA technique (rELISA) for quantifying IgG4 antibodies specific against the main allergens of *D*. *pteronyssinus,* more specifically Der p 1 or Der p 2, was compared to the conventional ELISA technique (cELISA) for quantifying IgG4 antibodies specific against the total extract from *D*. *pteronyssinus* (Dpt), as previously described, with modifications (Mastrandrea et al., Allergy, v.52, pages 1115-1119, 1997; Silva et al., Annals of Allergy, Asthma & Immunology, v.86, pages 545-550, 2001).

In order to perform this comparision, microtitration plates for high affinity ELISA were sensitized by the addition of 50 µL/well of allergens such as, for example, total extract of *D*. *pteronyssinus* (Dpt) in a protein concentration of 10 to 100 µg/mL or 100 to 5,000 Allergenic Unity per milliliter (AU/mL), preferentially 40 µg/mL or 1,500 AU/mL, diluted in a 0.06 M carbonate-bicarbonate buffer, pH 9.6, during 18 hours at 4°C.

Subsequently, the plates were washed 3 times with 0.01 M phosphate buffered saline (PBS), pH 7.2, containing 0.05% Tween 20 (PBS-T). After that, the plates' active sites, i.e. sites free from allergen binding, were blocked by addition of 200 µL/well of PBS-T during 1 hour at approximately room temperature, 20 to 25°C. Subsequent steps were performed using PBS-T-BSA as a diluent, ELISA buffer.

After the blocking of the active sites, in order to search for primary antibodies, the wells were washed as described above and subsequently incubated with 50 µL/well of serum samples of allergic patients diluted in a 1:5 ratio (in duplicate) during 1 hour at 37°C. The wells were washed 5 times and incubated with 50 µL/well of goat human-biotinylated secondary antibody anti-IgG4 diluted to 1:3000 during 1 hour at 37°C.

Subsequent steps involving the addition of straptavidin-peroxidase conjugate and of the enzymatic substrate, ABTS and hydrogen peroxide were performed in a similar manner as described for reverse ELISA. Results were expressed as ELISA unity per milliliter (EU/mL) or ELISA index (EI) obtained as described for reverse ELISA.

The specificity of the cELISA and rELISA assays was evaluated employing inhibition tests. Total allergenic extracts of *Dermatophagoides pteronyssinus* were submitted to serial decimal dilutions from 15,000 to 0.15 AU/mL in ELISA buffer.

Each extract dilution was added to a pool of reference human sera containing specific IgG4 antibodies diluted 1:2 to 1:50, preferentially 1:5, and afterwards incubated at 37°C during 2 hours. The pool of reference sera incubated only with the buffer was used as positive control.

IgG4 antibodies specific against Der p 1 or Der p 2 were determined by measuring the absorbance of both ELISAs as previously described. The inhibition percentage was calculated as follows: [1.0 - (test sample absorbance / positive control absorbance)] x 100.

Sensitivity curves of both ELISAs assays for the determination of the levels of IgG4 antibodies specific against the total extract of *D. pteronyssinus* (Dpt) and to the allergens Der p 1 and Der p 2, were illustrated in FIG.2.

In this particular example the detection limits of each assay were: 15.6 EU/mL for cELISA-Dpt, 1.9 EU/mL for rELISA-Der p 1 and 7.8 EU/mL for rELISA-Der p 2. The medium variation coefficients calculated for each dilution of the pool of analyzed reference sera were: 2.9%, 95% IC = 1.9 - 3.9% for cELISA-Dpt, 1.9%, 95% IC = 0.9 - 2.9% for rELISA-Der p 1 and 3.7%, 95% IC = 1.4 - 6.0% for rELISA-Der p 2.

Specificities of the assays for the detection of specific IgG4 antibodies against total extract of Dpt performed by cELISA and against Der p 1 and Der p 2 allergens performed by rELISA can be confirmed through inhibition tests, as shown in FIG. 3.

In this example, all the assays were inhibited in a dose-dependent manner, where a pool of reference sera containing specific IgG4 antibodies was incubated with increasing concentrations, 0.15 to 15,000 AU/mL, of total extract of *D*. *pteronyssinus.* Inhibition was higher than 80% for all assays, 88% for cELISA-Dpt, 82% for rELISA-Der p 1 and 89% for rELISA-Der p 2, in the highest concentration of dust mite extract, i.e. 15,000 AU/mL. However, the 50% inhibition doses (ID₅₀) were smaller for the cELISA-Dpt assays, with ID₅₀ = 14.42 AU/mL, and rELISA-Der p 2, ID₅₀ = 30.8 AU/mL, when compared to rELISA-Der p 1, ID₅₀ = 562,25 AU/mL, indicating that IgG4 antibodies specific against Der p 1 allergen were present in a higher concentration in the serum samples analyzed.

FIG. 4 illustrates the levels of IgG4 antibodies specific against the total extract of Dpt as determined by cELISA and against the allergens Der p 1 and Der p 2 as determined by rELISA in serum samples of two groups of patients: 15 patients allergic to dust mite under specific immunotherapy with the *D. pteronyssinus* extract (FIG. 4A: DPT group) and 15 patients without active immunotherapy (FIG. 4B: Placebo group) at two different times, day 0 and after one year of treatment.

Statistically significant differences before and after immunotherapy within each groups were determined by the Wilcoxon test (P < 0.05).

In the DPT group (FIG. 4A), there was a significant increase in levels of IgG4 antibodies specific against the total extract of Dpt, 1.9 vs 4.8; P = 0.0034, and against the Der p 1 allergen, 1.7 *vs* 4.1; P = 0.0215, but not to the Der p 2 allergen, 2.2 *vs* 5.3; P > 0.05.

On the other hand, no significant alteration was observed in levels of IgG4 antibodies specific against the the total extract of Dpt, 1.3 vs 1.1; P > 0.05, Der p 1, 1.0 vs 1.0; P > 0.05 or Der p 2, 2.3 *vs* 1.5; P > 0.05 in the Placebo group after one year of treatment (FIG. 4B).

Thus, in view of what was described above, reverse ELISA has been shown to be a sensitive and alternative method for quantifying seric allergen-specific IgG4 antibodies, providing valuable information for the control and attendance of specific immunotherapy with peptides or native or recombinant allergens of clinical importance.

Modifications and variations of the technique described in the present invention that may be apparent to those skilled in the art, will remain as part of the scope of the present invention.

## Claims

1. Method for measuring allergen-specific human IgG antibody subclasses for monitoring patients with allergic respiratory disease under allergen-specific immunotherapy using an immunoenzymatic technique comprising the following steps:
• Sensitization of high affinity microtitration plates for ELISA with mouse monoclonal antibodies against specific allergens;
• Blocking of the active sites of high affinity microtitration plates for ELISA with PBS-T-BSA;
• Addition of crude extract from the corresponding allergen;
• Incubation with serum samples or other biological fluids from allergic patients;
• Addition of labeled secondary mouse monoclonal antibodies anti-human IgG1, anti-human IgG2, anti-human IgG3 or anti-human IgG4;
• Addition of an enzymatic conjugate;
• Addition of an enzymatic substrate diluted in a chromogenic buffer;
• Determination of absorbance in a microtitration plate reader; and
• Expression of results in EU/mL compared to a standard curve obtained using reference human sera.

2. Method according to claim 1, wherein mouse monoclonal antibody clones against anti-Der p 1 or against dust mite allergens such as Der p 2, Der f 1, Der f 2, Blo t 5 are used in the initial step of the reaction.

3. Method according to claim 2, wherein the mouse monoclonal antibodies against specific allergens for coating high affinity microtitration plates for ELISA are selected from a group consisting of, but not limited to anti-Der p 1, 5H8 clone and anti-Der p 2, 1D8 clone.

4. Method according to anyone of the claims 1 to 3, wherein, in the step concerning sensitization of high affinity microtitration plates for ELISA, 0.5 to 5µg/well of mouse monoclonal antibody against specific allergens are added to each well, diluted in 0.01 M to 0.1 M carbonate-bicarbonate buffer, pH 7.0 to 12.0, and incubated during 6 to 24 hours at a temperature of 2 to 12°C, wherein preferably 1 µg/well of mouse monoclonal antibody against specific allergens are added to each well, diluted in 0.06 M carbonate-bicarbonate buffer, pH 9.6, and incubated during 18 hours at 4°C.

5. Method according to claim 1, wherein in the step concerning the blocking of the active sites of microtitration plates with PBS-T-BSA, the active sites are blocked by the addition of 50 to 300 µL/well of PBS-T containing 0.5 to 5% bovine serum albumin (BSA), and incubated during 30 minutes to 4 hours at a temperature of 20°C to 40°C, wherein preferably 200 µL of PBS-T and 1% BSA are added to each well and incubated during 1 hour at room temperature or at 37°C.

6. Method according to claim 1, wherein in the step concerning addition of crude extract from the corresponding allergen, crude extracts from dust mite allergens are used, wherein the corresponding allergen crude extract preferably is from Dermatophagoides pteronyssinus..

7. Method according to claim 6, wherein the wells are incubated with 20 to 200 µL/well of the crude extract from the corresponding allergen in a protein concentration from 10 to 100 µg/mL or 100 to 5,000 Allergenic Units per milliliter (AU/mL), during 30 minutes to 4 hours at a temperature from 20°C to 40°C, wherein preferably 50 µL/well of the crude extract from the corresponding allergen are added in a concentration of 1,500 AU/mL or 40µg/mL and incubated during 1 hour at room temperature or at 37°C.

8. Method according to claim 1, wherein in the step concerning incubation with serum samples or other biological fluids from allergic patients, the plates are incubated during 30 minutes to 4 hours at a temperature from 20°C to 40°C with 25 to 1.00 µL/well of serum samples or other biological fluid samples of allergic patients, primary antibodies, from undiluted up to 1:20 diluted samples, wherein the plates preferably were incubated during 1 hour with 50 µL/well of serum samples or other biological fluid samples from allergic patients, in dilutions of 1:2 and 1:5.

9. Method according to claim 1, wherein in the step concerning addition of labeled secondary mouse monoclonal antibodies anti-human IgG1, anti-human IgG2, anti-human IgG3 or anti-human IgG4, the label is selected from a group consisting of, but not limited to biotin or peroxidase, wherein the label preferably is biotin.

10. Method according to claim 9, wherein the plates are incubated during 30 minutes to 4 hours at a temperature from 20°C to 40°C with 25 to 100 µL/well of labelled secondary mouse monoclonal antibodies anti-human IgG1, anti-human IgG2, anti-human IgG3 or anti-human IgG4, in a dilution from 1:100 to 1:6,000, wherein the plates preferably are incubated during 1 hour at room temperature or at 37°C with 50µL/well of biotinylated secondary mouse monoclonal antibody anti-human IgG4 in a dilution of 1:3,000.

11. Method according to claim 1, wherein in the step concerning addition of an enzymatic conjugate, the enzymatic conjugate is , preferentially, streptavidin-peroxidase, wherein the plates preferably are incubated during 15 minutes to 4 hours at a temperature from 20°C to 40°C with 25 to 200 µL/well of streptavidin-peroxidase in a dilution from 1:100 to 1:300, wherein the plates more preferably are incubated during 30 minutes at room temperature or at 37°C with 50 µL/well of streptavidin-peroxidase in a dilution of 1:500.

12. Method according to claim 1, wherein in the step concerning addition of an enzymatic substrate diluted in a chromogenic buffer, the enzymatic substrate is hydrogen peroxide, and the chromogenic buffer is selected from a group consisting of , but not limited to 2,2'-azino-bis (3-ethylbenzthiazoline-6-sulphonic acid) (ABTS), orthophenilenediamine (OPD) or tetramethylbenzidine (TMB), wherein the chromogenic buffer preferably is ABTS.

13. Method according to claim 12, wherein 25 to 200 µL/well of hydrogen peroxide are added in a concentration from 0.01 to 0.1% in 0.01 to 0.1 M chromogenic buffer diluted from 0.01 to 0.1 M citrate-phosphate buffer, pH between 4.0 and 5.0, wherein preferably 50 µL/well of hydrogen peroxide are added in a concentration of 0.03% in 0.01 M ABTS diluted in 0.07 M citrate-phosphate buffer at pH 4.2.

14. Method according to claim 1, wherein in the step concerning determination of absorbance in a microtitration plate reader, wavelengths of 405, 450 or 492 nm are used, depending on the chromogenic buffer employed in the step concerning the addition of an enzymatic substrate diluted in a chromogenic buffer, wherein the wavelength used preferably is 405 nm.

15. Method according to claim 1, wherein in the step concerning expression of results in EU/mL compared with a standard curve using reference human sera, the standard curve is obtained using known quantities of human IgG antibody subclasses, particularly IgG1, IgG2, IgG3 or IgG4 against purified immunodominant allergens, or IgG antibodies, more specifically chimeric IgG1, IgG2, IgG3 or IgG4 constituted by human Fc gamma chains conjugated to Fab portions, an antigen binding fragment, against immunodominant allergen produced in animals, preferentially mice, wherein the standard curve preferably is achieved using known quantities of purified IgG antibodies against Der p 1 or Der p 2 or anti-Der p 2 chimeric IgG antibodies constituted by human Fc gamma chains conjugated to Fab portions, an antigen binding fragment, against immunodominant allergens produced in mice.

## Patentansprüche

1. Verfahren zur Messung allergenspezifischer humaner IgG-Antikörperunterklassen zur Überwachung von Patienten mit einer allergischen Atemwegserkrankung unter allergenspezifischer Immuntherapie mittels einer immunenzymatischen Methode, wobei das Verfahren die folgenden Schritte umfasst:
• Sensibilisieren hochaffiner Mikrotiterplatten für einen ELISA mit monoklonalen Mausantikörpern gegen spezifische Allergene;
• Blockieren der aktiven Zentren hochaffiner Mikrotiterplatten für einen ELISA mit PBS-T-BSA;
• Hinzufügen eines Rohextrakts des entsprechenden Allergens;
• Inkubieren mit Serumproben oder anderen biologischen Flüssigkeiten von allergischen Patienten;
• Hinzufügen markierter sekundärer monoklonaler Mausantikörper Anti-Human-IgG1, Anti-Human-IgG2, Anti-Human-IgG3 oder Anti-Human-IgG4;
• Hinzufügen eines Enzym-Konjugats;
• Hinzufügen eines in einem chromogenen Puffer verdünnten Enzym-Substrats;
• Feststellen der Absorbanz in einem Mikrotiterplatten-Lesegerät; und
• Ausdrücken der Ergebnisse in EU/mL verglichen mit einer anhand humaner Referenzseren ermittelten Standardkurve.

2. Verfahren nach Anspruch 1, wobei im ersten Schritt der Reaktion monoklonale Mausantikörperklone gegen anti-Der p 1 oder gegen Hausstaubmilbenallergene wie z.B. Der p 2, Der f 1, Der f 2, Blo t 5 verwendet werden.

3. Verfahren nach Anspruch 2, wobei die monoklonalen Mausantikörper gegen spezifische Allergene zur Beschichtung hochaffiner Mikrotiterplatten für einen ELISA aus einer Gruppe bestehend aus, jedoch nicht beschränkt auf anti-Der p 1, Klon 5H8 und anti-Der p 2, Klon 1D8 ausgewählt sind.

4. Verfahren nach einem der Ansprüche 1 bis 3, wobei in dem Schritt des Sensibilisierens hochaffiner Mikrotiterplatten für einen ELISA 0,5 bis 5 µg/Well monoklonaler Mausantikörper gegen spezifische Allergene in jeden Well gegeben wird, verdünnt in 0,01 M bis 0,1 M Carbonat-Bicarbonat-Puffer, pH 7,0 bis 12,0, und für 6 bis 24 Stunden bei einer Temperatur von 2 bis 12 °C inkubiert wird, wobei vorzugsweise 1 µg/Well monoklonaler Mausantikörper gegen spezifische Allergene in jeden Well gegeben wird, verdünnt in 0,06 M Carbonat-Bicarbonat-Puffer, pH 9,6, und für 18 Stunden bei 4 °C inkubiert wird.

5. Verfahren nach Anspruch 1, wobei in dem Schritt des Blockierens der aktiven Zentren von Mikrotiterplatten mit PBS-T-BSA die aktiven Zentren durch Hinzufügen von 50 bis 300 µL/Well PBS-T enthaltend 0,5 bis 5 % bovines Serumalbumin (BSA) und Inkubieren für 30 Minuten bis 4 Stunden bei einer Temperatur von 20 °C bis 40 °C blockiert werden, wobei vorzugsweise 200 µL PBS-T und 1 % BSA in jeden Well gegeben und für 1 Stunde bei Raumtemperatur oder bei 37 °C inkubiert werden.

6. Verfahren nach Anspruch 1, wobei in dem Schritt des Hinzufügens eines Rohextrakts des entsprechenden Allergens Rohextrakte von Hausstaubmilbenallergenen verwendet werden, wobei der entsprechende Allergenrohextrakt vorzugsweise von *Dermatophagoides pteronyssinus* stammt.

7. Verfahren nach Anspruch 6, wobei die Wells mit 20 bis 200 µL/Well des Rohextrakts des entsprechenden Allergens in einer Proteinkonzentration von 10 bis 100 µg/mL oder 100 bis 5000 Allergeneinheiten pro Milliliter (AU/mL) für 30 Minuten bis 4 Stunden bei einer Temperatur von 20 °C bis 40 °C inkubiert werden, wobei vorzugsweise 50 µL/Well des Rohextrakts des entsprechenden Allergens in einer Konzentration von 1500 AU/mL oder 40 µg/mL hinzugefügt und für 1 Stunde bei Raumtemperatur oder bei 37 °C inkubiert werden.

8. Verfahren nach Anspruch 1, wobei in dem Schritt des Inkubierens mit Serumproben oder anderen biologischen Flüssigkeiten allergischer Patienten die Platten für 30 Minuten bis 4 Stunden bei einer Temperatur von 20 °C bis 40 °C mit 25 bis 100 µL/Well Serumproben oder anderen biologischen Flüssigkeitsproben allergischer Patienten, primären Antikörpern, aus unverdünnten bis zu 1:20 verdünnten Proben inkubiert werden, wobei die Platten vorzugsweise für 1 Stunde mit 50 µL/Well Serumproben oder anderen biologischen Proben allergischer Patienten in Verdünnungen von 1:2 und 1:5 inkubiert wurden.

9. Verfahren nach Anspruch 1, wobei in dem Schritt des Hinzufügens markierter sekundärer monoklonaler Mausantikörper Anti-Human-IgG1, Anti-Human-IgG2, Anti-Human-IgG3 oder Anti-Human-IgG4, der Marker aus einer Gruppe bestehend aus, jedoch nicht beschränkt auf Biotin oder Peroxidase ausgewählt ist, wobei es sich bei dem Marker vorzugsweise um Biotin handelt.

10. Verfahren nach Anspruch 9, wobei die Platten für 30 Minuten bis 4 Stunden bei einer Temperatur von 20 °C bis 40 °C mit 25 bis 100 µL/Well markierten sekundären monoklonalen Mausantikörpern Anti-Human-IgG1, Anti-Human-IgG2, Anti-Human-IgG3 oder Anti-Human-IgG4, in einer Verdünnung von 1:100 bis 1:6000 inkubiert werden, wobei die Platten vorzugsweise für 1 Stunde bei Raumtemperatur oder bei 37 °C mit 50 µL/Well biotinyliertem sekundärem monoklonalem Mausantikörper Anti-Human-IgG4 in einer Verdünnung von 1:3000 inkubiert werden.

11. Verfahren nach Anspruch 1, wobei in dem Schritt des Hinzufügens eines Enzym-Konjugats es sich bei dem Enzym-Konjugat vorzugsweise um Streptavidin-Peroxidase handelt, wobei die Platten vorzugsweise für 15 Minuten bis 4 Stunden bei einer Temperatur von 20 °C bis 40 °C mit 25 bis 200 µL/Well Streptavidin-Peroxidase in einer Verdünnung von 1:100 bis 1:300 inkubiert werden, wobei die Platten noch bevorzugter für 30 Minuten bei Raumtemperatur oder bei 37 °C mit 50 µL/Well Streptavidin-Peroxidase in einer Verdünnung von 1:500 inkubiert werden.

12. Verfahren nach Anspruch 1, wobei in dem Schritt des Hinzufügens eines in einem chromogenen Puffer verdünnten Enzym-Substrats es sich bei dem Enzym-Substrat um Wasserstoffperoxid handelt und der chromogene Puffer aus einer Gruppe bestehend aus, jedoch nicht beschränkt auf 2,2'-Azino-bis-(3-ethylbenzthiazolin-6-sulfonsäure) (ABTS), ortho-Phenylendiamin (OPD) oder Tetramethylbenzidin (TMB) ausgewählt ist, wobei es sich bei dem chromogenen Puffer vorzugsweise um ABTS handelt.

13. Verfahren nach Anspruch 12, wobei 25 bis 200 µL/Well Wasserstoffperoxid in einer Konzentration von 0,01 bis 0,1 % in 0,01 bis 0,1 M chromogenem Puffer verdünnt in 0,01 bis 0,1 M Citrat-Phosphat-Puffer, pH zwischen 4,0 und 5,0, hinzugefügt werden, wobei vorzugsweise 50 µL/Well Wasserstoffperoxid in einer Konzentration von 0,03 % in 0,01 M ABTS verdünnt in 0,07 M Citrat-Phosphat-Puffer bei pH 4,2 hinzugefügt werden.

14. Verfahren nach Anspruch 1, wobei in dem Schritt des Bestimmens der Absorbanz in einem Mikrotiterplatten-Lesegerät, abhängig von dem im Schritt des Hinzufügens eines in einem chromogenen Puffer verdünnten Enzym-Substrats verwendeten chromogenen Puffer, Wellenlängen von 405, 450 oder 492 nm eingesetzt werden, wobei die eingesetzte Wellenlänge vorzugsweise 405 nm beträgt.

15. Verfahren nach Anspruch 1, wobei in dem Schritt des Ausdrückens der Ergebnisse in EU/mL verglichen mit einer anhand humaner Referenzseren ermittelten Standardkurve die Standardkurve anhand von bekannten Mengen humaner IgG-Antikörperunterklassen, insbesondere IgG1, IgG2, IgG3 oder IgG4 gegen aufgereinigte immundominante Allergene, oder IgG-Antikörpern, insbesondere aus mit Fab-Abschnitten, einem antigenbindenden Fragment, konjugierten humanen Fc-Gamma-Ketten gebildeten chimären IgG1, IgG2, IgG3 oder IgG4 gegen in Tieren, vorzugsweise Mäusen, erzeugte immundominante Allergene erlangt wird, wobei die Standardkurve vorzugsweise anhand von bekannten Mengen aufgereinigter IgG-Antikörper gegen chimäre IgG Antikörper Der p 1 oder Der p 2 oder anti-Der p 2, gebildet von mit Fab-Abschnitten, einem antigenbindenden Fragment, konjugierten humanen Fc-Gamma-Ketten, gegen in Mäusen erzeugte immundominante Allergene erlangt wird.

## Revendications

1. Procédé de mesure de sous-classes d'anticorps IgG humains spécifiques d'allergènes pour la surveillance de patients ayant une maladie respiratoire allergique sous immunothérapie spécifique d'un allergène utilisant une technique immunoenzymatique comprenant les étapes suivantes :
• sensibiliser des plaques microtitre à haute affinité pour un ELISA par des anticorps monoclonaux de souris contre des allergènes spécifiques ;
• bloquer les sites actifs des plaques microtitre à haute affinité pour un ELISA par PBS-T-BSA ;
• ajouter un extrait brut de l'allergène correspondant ;
• incuber avec des échantillons de sérum ou d'autres fluides biologiques de patients allergiques ;
• ajouter des anticorps monoclonaux de souris secondaires marqués IgG1 anti-humaine, IgG2 anti-humaine, IgG3 anti-humaine ou IgG4 anti-humaine ;
• ajouter un conjugué enzymatique ;
• ajouter un substrat enzymatique dilué dans un tampon chromogène ;
• déterminer l'absorbance dans un lecteur de plaques microtitre ; et
• exprimer les résultats en EU/mL par rapport à une courbe standard obtenue en utilisant des sérums humains de référence.

2. Procédé selon la revendication 1, dans lequel des clones d'anticorps monoclonaux de souris contre anti-Der p 1 ou contre des allergènes d'acariens tels que Der p 2, Der f 1, Der f 2, Blo t 5 sont utilisés dans la première étape de la réaction.

3. Procédé selon la revendication 2, dans lequel les anticorps monoclonaux de souris contre des allergènes spécifiques pour recouvrir des plaques microtitre à haute affinité pour un ELISA sont sélectionnés dans un groupe consistant en, sans s'y limiter, anti-Der p 1, clone 5H8 et anti-Der p 2, clone 1D8.

4. Procédé selon l'une quelconque des revendications 1 à 3, dans lequel, dans l'étape concernant la sensibilisation des plaques microtitre à haute affinité pour un ELISA, 0,5 à 5 µg/puits d'anticorps monoclonal de souris contre des allergènes spécifiques sont ajoutés à chaque puits, dilués dans 0,01 M à 0,1 M de tampon carbonate-bicarbonate de pH 7,0 à 12,0 et sont incubés pendant 6 à 24 heures à une température de 2 à 12 °C, dans lequel, de préférence, 1 µg/puits d'anticorps monoclonal de souris contre des allergènes spécifiques sont ajoutés à chaque puits, dilués dans 0,06 M de tampon carbonate-bicarbonate de pH 9,6 et sont incubés pendant 18 heures à 4 °C.

5. Procédé selon la revendication 1, dans lequel, dans l'étape concernant le blocage des sites actifs de plaques microtitre par PBS-T-BSA, les sites actifs sont bloqués en ajoutant 50 à 300 µL/puits de PBS-T contenant 0,5 à 5 % d'albumine de sérum bovin (BSA) et sont incubés pendant 30 minutes à 4 heures à une température de 20 °C à 40 °C, dans lequel, de préférence, 200 µL de PBS-T et 1% de BSA sont ajoutés à chaque puits et sont incubés pendant 1 heure à température ambiante ou à 37 °C.

6. Procédé selon la revendication 1, dans lequel, dans l'étape concernant l'ajout de l'extrait brut de l'allergène correspondant, des extraits bruts des allergènes d'acariens sont utilisés, dans lequel, de préférence, l'extrait brut d'allergène correspondant est de *Dermatophagoides pteronyssinus.*

7. Procédé selon la revendication 6, dans lequel les puits sont incubés avec 20 à 200 µL/puits de l'extrait brut de l'allergène correspondant dans une concentration de protéines de 10 à 100 µg/mL ou 100 à 5000 Allergenic Units par millilitre (AU/mL) pendant 30 minutes à 4 heures à une température de 20 °C à 40 °C, dans lequel, de préférence, 50 µL/puits de l'extrait brut de l'allergène correspondant sont ajoutés dans une concentration de 1500 AU/mL ou 40 µg/mL et sont incubés pendant 1 heure à température ambiante ou à 37 °C.

8. Procédé selon la revendication 1, dans lequel, dans l'étape concernant l'incubation avec des échantillons de sérum ou d'autres fluides biologiques de patients allergiques, les plaques sont incubées pendant 30 minutes à 4 heures à une température de 20 °C à 40 °C avec 25 à 100 µL/puits d'échantillons de sérum ou d'autres échantillons de fluides biologiques de patients allergiques, des anticorps primaires, d'échantillons non dilués à dilués en 1:20, dans lequel, de préférence, les plaques ont été incubées pendant 1 heure avec 50 µL/puits d'échantillons de sérum ou d'autres échantillons de fluides biologiques de patients allergiques dans des dilutions de 1:2 et 1:5.

9. Procédé selon la revendication 1, dans lequel, dans l'étape concernant l'ajout d' anticorps monoclonaux de souris secondaires marqués IgG1 anti-humaine, IgG2 anti-humaine, IgG3 anti-humaine ou IgG4 anti-humaine, le marqueur est sélectionné dans un groupe consistant en, sans s'y limiter, la biotine ou la peroxydase, dans lequel, de préférence, le marqueur est la biotine.

10. Procédé selon la revendication 9, dans lequel les plaques sont incubées pendant 30 minutes à 4 heures à une température de 20 °C à 40 °C avec 25 à 100 µL/puits d'anticorps monoclonaux de souris secondaires marqués IgG1 anti-humaine, IgG2 anti-humaine, IgG3 anti-humaine ou IgG4 anti-humaine dans une dilution de 1:100 à 1:6000, dans lequel, de préférence, les plaques sont incubées pendant 1 heure à température ambiante ou à 37 °C avec 50 µL/puits d'anticorps monoclonal de souris secondaire biotinylé IgG4 anti-humaine dans une dilution de 1:3000.

11. Procédé selon la revendication 1, dans lequel, dans l'étape concernant l'ajout d'un conjugué enzymatique, de préférence, le conjugué enzymatique est la streptavidine-peroxydase, dans lequel, de préférence, les plaques sont incubées pendant 15 minutes à 4 heures à une température de 20 °C à 40 °C avec 25 à 200 µL/puits de streptavidine-peroxydase dans une dilution de 1:100 à 1:300, dans lequel, de manière plus préférée, les plaques sont incubées pendant 30 minutes à température ambiante ou à 37 °C avec 50 µL/puits de streptavidine-peroxydase dans une dilution de 1:500.

12. Procédé selon la revendication 1, dans lequel, dans l'étape concernant l'ajout d'un substrat enzymatique dilué dans un tampon chromogène, le substrat enzymatique est le peroxyde d'hydrogène, et le tampon chromogène est sélectionné dans un groupe consistant en, sans s'y limiter, le 2,2'- azino-bis-(3-éthylbenzthiazoline-6-acide sulfonique) (ABTS), l'orthophénilènediamine (OPD) ou le tétraméthylbenzidine (TMB), dans lequel, de préférence, le tampon chromogène est l'ABTS.

13. Procédé selon la revendication 12, dans lequel 25 à 200 µL/puits de peroxyde d'hydrogène sont ajoutés dans une concentration de 0,01 à 0,1 % dans 0,01 à 0,1 M de tampon chromogène dans une dilution de 0,01 à 1 M de tampon citrate-phosphate de pH entre 4,0 et 5,0, dans lequel, de préférence, 50 µL/puits de peroxyde d'hydrogène sont ajoutés dans une concentration de 0,03 % dans 0,01 M d'ABTS dilué dans 0,07 M de tampon citrate-phosphate de pH 4,2.

14. Procédé selon la revendication 1, dans lequel, dans l'étape concernant la détermination de l'absorbance dans un lecteur de plaques microtitre, des longueurs d'onde de 405, 450 ou 492 nm sont utilisées en fonction du tampon chromogène employé dans l'étape concernant l'ajout d'un substrat enzymatique dilué dans un tampon chromogène, dans lequel, de préférence, la longueur d'onde utilisée est 405 nm.

15. Procédé selon la revendication 1, dans lequel, dans l'étape concernant l'expression des résultats en EU/mL par rapport à une courbe standard utilisant des sérums humains de référence, la courbe standard est obtenue en utilisant des quantités connues de sous-classes d'anticorps IgG humain, en particulier IgG1, IgG2, IgG3 ou IgG4 contre des allergènes immunodominants purifiés, ou d'anticorps IgG, plus particulièrement l'IgG1, IgG2, IgG3 ou IgG4 chimérique constituée par des chaînes gamma Fc humaines conjuguées à des parties Fab, un fragment de liaison d'antigène, contre des allergènes immunodominants produits dans les animaux, de préférence des souris, dans lequel, de préférence, la courbe standard est obtenue en utilisant des quantités connues d'anticorps IgG purifiés contre Der p 1 ou Der p 2 ou anticorps IgG chimériques anti-Der p 2 constitués par chaînes gamma Fc humaines conjuguées à des parties Fab, un fragment de liaison d'antigène, contre des allergènes immunodominants produits dans les souris.
